# EUROPÄISCHE PATENTSCHRIFT

(11) **EP 1 318 851 B1**
(45) Veröffentlichungstag und Bekanntmachung des Hinweises auf die Patenterteilung: **23.11.2005**
(21) Anmeldenummer: 01982228.7
(22) Anmeldetag: 31.08.2001
(51) Int. Cl.: A61M 16/08

(54) **Vorrichtung zum Trennen von Gas- und Flüssigkeiten, insbesondere an Luftröhrenkathetern**
Device for separating gas and liquid streams, especially in tracheal catheters
Dispositif pour séparer des flux de gaz et de liquide, notamment dans des sondes d'aspiration trachéales

(30) Priorität: 19.09.2000 DE 10046196
(43) Veröffentlichungstag der Anmeldung: 18.06.2003
(73) Patentinhaber: Vogt, Hendrik, 90596 Schwanstetten (DE)
(72) Erfinder: Vogt, Hendrik, 90596 Schwanstetten (DE)
(74) Vertreter: Lösch, Christoph Ludwig Klaus
(86) Internationale Anmeldenummer: PCT/EP2001/010054
(87) Internationale Veröffentlichungsnummer: WO 2002/024270

(56) Entgegenhaltungen:
- WO-A-97/47345
- DE-C- 19 838 370
- DE-U- 20 016 171
- US-A- 4 507 120
- US-A- 5 195 664

## Beschreibung

Die Erfindung betrifft eine Vorrichtung zum Trennen von Gas- und Flüssigkeitsströmen, insbesondere an Luftröhrenkathetern von Patienten mit einem Kanäle für die Zu- und Abführung der Atemluft und der Abführung von Sputum aufweisenden Gehäuse.

Bei einer bekannten Vorrichtung (U.S.-PS 5765 557) dieser Art ist bei Patienten mit Luftröhrenkathetern im Inneren ein Kanal mit Pfaden für die Zu- und Abführung der Atemluft eingesetzt und vermittels einer Steuervorrichtung über den Abführungskanal für die Atemluft durch die Kraft von Atemluft anfallendes Sputum entfernbar. Die Vorrichtung ist dabei von außen nicht einsehbar und wird wegen der Nichteinsehbarkeit der Funktion als nachteilig empfunden. Weiter ist es bekannt (DE 198 38 370 C1) an dem Luftröhrenkatheter eine Vorrichtung anzubringen, bei der durch ein Ventil eine zusätzliche Öffnung kontrollierbar ist, über die das Sputum durch abströmende Atemluft entfernbar ist, wobei das Ventil zum Abfluß des Sputums beim Ausatmungsvorgang öffnet und während des Einatmens geschlossen ist. Von Nachteil ist bei dieser Vorrichtung, daß bei Lageänderungem des Patienten, z.B. aus einer aufrechten Stellung oder aus der Rückenlage das Sputum die ventilgesteuerte Öffnung nicht erreicht und nicht abgeführt werden kann,während das nichtabführbare Sputum vielfach zu Verstopfungen der für die Zu- und Abführung der Atemluft vorgesehenen Öffnungen führt. Die Entfernung des Sputums bedarf deshalb bei wechselnden Lagen des Patienten jeweils Nachführbewegungen der Vorrichtung, was einen hohen Arbeitsaufwand für das Pflegepersonal erfordert und für den Patienten unangenehm und schmerzhaft ist.

Aufgabe der Erfindung ist es unter Belassung einer behinderungsfreien Zu- und Abführung der Atemluft Maßnahmen zum sicheren Abführen von Sputum unter dem Einfluß der Atemluft unabhängig der jeweiligen Lage des Patienten zu schaffen.

Der Erfindung gemäß ist diese Aufgabe durch die Anordnung eines mindestens kugelabschnittsförmigen geschlossenen Gehäuses mit im Endflächenbereich des Gehäuses im Abstand voneinander angeordneten rohrförmigen Kanälen zu je einer Verbindung derselben mit dem Luftröhrenkatheter und zur Durchleitung von Atemluft sowie Bildung eines rohrförmigen Kanals zur Abführung von Sputum nach außen, dessen inneres Stirnende verschlossen ist und an dessen Kanalinnenraum ein durch Schwerkraft selbständig abbiegbarer und/oder abschwenkbarer rohrförmiger Rüssel aus einem hoch flexiblen oder federnd elastischen Werkstoff anliegt, der quer zum Sputumkanal sich erstreckend ausgebildet ist und mit seinem freien Ende im Abstand bis annähernd der Innenwand des Gehäuses reicht. Bei der Vorrichtung bleiben die der Zu- und Abführung dienenden Kanäle permanent behinderungsfrei offen, während bei Hustenvorgängen od. dgl. Sputum zunächst in das Gehäuse übertritt, sich in der bodenseitigen Kugelzone sammelt um von dort durch den Atemdruck und/oder ausgestoßenen Hustendruck über den Rüssel in den Kanal für das Sputum zu gelangen und über diesem abgeführt zu werden. Die kugelige Form des Gehäuses gewährleistet dabei unabhängig der Stellung oder Lage des Patienten stets die Bildung einer als Sumpf wirkenden und das Sputum aufnehmenden bodenseitigen Kugelzone, der sich der hoch flexible Rüssel jeweils mit seinem freien Ende selbsttätig zuordnet.

Bevorzugt ist das Gehäuse der Vorrichtung als Halb- oder Vollkugel ausgebildet, wobei sich die Kanäle im Endflächenbereich oder dem Bereich der durch den Kugelmittelpunkt geführten Ebene erstrecken. Bei kugelabschnittsförmigen Gehäusen kann auf der Endfläche ein unabhängiger Aufsatz vorgesehen sein, der die Kanäle aufnimmt und von dem aus über eine Durchtrittslochung in der Trennwand von Aufsatz und Gehäuse der Rüssel in das Gehäuse über seine Länge abbieg- oder abschwenkbar einragt und im Abstand bis annähernd der Innenwand des Gehäuses geführt ist. Der Rüssel kann fest mit dem Kanal zur Abführung des Sputums in Verbindung stehen oder gemäß vorteilhafter Ausbildung auf dem Kanal um die Längsachse desselben frei drehbar dicht ausgebildet sein. Zweckmäßig kann der Rüssel hierzu mit seinem kanalseitigen Ende in einen topfförmigen Zylinder einmünden und vermittels des Zylinders auf dem Sputumkanal frei drehbar sein.

Vorteilhafte Ausgestaltungen der Vorrichtung sind durch die Maßnahmen der Ansprüche 7 bis 22 bestimmt. So kann der Rüssel mit seinem freien Ende auch an der Innenwand des Gehäuses anliegen, wobei zur Sicherung der Einleitung von Sputum in den Rüssel am gehäuseseitigen Ende desselben Durchführungsöffnungen, wie Aussparungen, Schlitzungen od.dgl. erforderlich sind. Ferner kann der Rüssel am freien Ende einen Körper mit großem spezifischen Gewicht tragen, der für eine selbständige radiale Ausrichtung von Rüssel bzw. Rüssel und topfförmigem Zylinder zum Kanal für die Sputum-Ableitung sorgt. Auch ist möglich, die Kanäle für die Abführung des Sputums und der Durchleitung der Atemluft insgesamt relativ zum Gehäuse verschwenk- oder verdrehbar auszuführen.

Eine besonders sichere Entfernung des Sputums ist erreichbar, wenn am Kanal für die Abführung des Sputums eine Pumpe anliegt, deren Eingang mit dem Rüssel und deren Ausgang jeweils mit dem Kanal verbunden ist. Eine solche Pumpe ist als Kolben- oder Membranpumpe ausbildbar, deren Kolben oder Membrane durch Atemdruck aus der Ruhestellung in die Förderstellung für das Sputum bringbar und bei Fehlen von Atemdruck vorzugsweise durch Feder- und/oder Schwerkraft in die Ruhestellung sebsttätig rückführbar sind und das Sputum bei geschlossenem Eingang in den Sputumkanal eindrückt. Die Federkraft ist dabei durch einen verformbaren Plattenkörper od.dgl. aus einem federnd elastischen Werkstoff oder vermittels an einer gewellten Manschette aus federnd elastischem Werkstoff, z.B. Gummi, angeordneten starren Plattenkörper aufbringbar, die jeweils über Betätigungsglieder, z.B. Stangen mit Kolben oder Membrane kraftschlüssig verbunden sind.

Auch hat sich die Anordnung einer Rückflußsperre für das Sputum im Sputumkanal als vorteilhaft erwiesen. Desweiteren ist eine Erhöhung des Hustendruckes im Gehäuse durch einen Schieber oder Klappe im Kanal für die Zuleitung der Atemluft zu erreichen, die den Kanal über einen Teilquerschnitt verdecken und durch abfließende Atemluft zur Freigabe eines größeren oder ganzen Querschnitts selbsttätig verschieb- oder verschwenkbar sind oder in Einatmungsrichtung öffnen.

Schließlich kann der Kanal für die Zuleitung der Atemluft eingangsseitig einen Luftfilter tragen, der gegebenenfalls mit einer elektrischen Einrichtung zur Vorwärmung der Atemluft und/oder mit Befeuchtungsmaßnahmen versehen ist.

Eine bevorzugte Ausführungsform der Vorrichtung ist dadurch erreichbar, wenn das Gehäuse durch je einen im Abstand einander zentrisch umfassenden Außen- und Innenkörper gebildet ist, deren Zwischenraum einen gehäuse- oder schwammförmigen Filterwerkstoff aufnimmt, der innenseitig zur Bildung eines umlaufenden Spaltenraums vermittels Abstandshalter mit Abstand zum Innenkörper durch eine gelochte Folie begrenzt und über eine Teillänge mit einer als Sammelraum wirkenden Aussparung versehen ist, an der die Zuleitung für die Atemluft anliegt und zur Einbringung von Zuluft in den Zwischenraum Durchflußöffnungen im Außenkörper aufweist.

Letztlich ist eine alternative Ausgestaltung der Vorrichtung durch ein zylindrisches, geschlossenes Gehäuse erzielt, das Kanäle für die Festlegung desselben am Luftröhrenkatheter, der Zu- und Abführung von Atemluft und Abführung des Sputums aufweist, wobei letzterer mit einem bis an oder annähernd der Gehäuseinnenwand reichenden Rüssel zur Aufnahme und Weiterleitung von Sputum in Verbindung steht.

Es versteht sich, daß die Vorrichtung in Abwandlung des Erfindungsgedankens auch zur Trennung von luftbelasteten Mineralölen, wie Altöl von Verbrennungskraftmotoren, in Anwendung kommen kann.

Die Erfindung ist in den Figuren verdeutlicht. Es zeigen:
- Fig. 1: eine Vorrichtung in Seitenansicht,
- Fig. 2: eine Vorrichtung gemäß Fig. 1, in Draufsicht,
- Fig. 3: einen Rüssel mit einem Teilstück des Sputumkanals, im Schnitt,
- Fig. 4: eine Vorrichtung mit Sputumpumpe, teilweise im Schnitt,
- Fig. 5: eine Sputumpumpe nach Fig. 4 in anderer Arbeitsstellung, im Schnitt
- Fig. 6: eine Vorrichtung abgewandelter Ausführung, im Schnitt,
- Fig. 7: einen Kanal zur Luftdurchführung mit Sperrglied, im Schnitt,
- Fig. 8: ein Sperrglied in Vorderansicht, vergrößert,
- Fig. 9: eine Rückflußsperre in Draufsicht, vergrößert,
- Fig.10: eine Rückflußsperre der Fig. 9 in Seitenansicht, vergrößert,
- Fig.11: einen Luftfilter im Schnitt, vergrößert,
- Fig.12: einen Rüssel mit einem Teilstück des Sputumkanals abgewandelter Ausführung, in Seitenansicht,
- Fig.13: einen Kanalabschnitt zur Durchleitung der Atemluft mit einem Sperrglied in Seitenansicht, vergrößert,
- Fig.14: ein Sperrglied gemäß Fig. 7 im Schnitt, vergößert,
- Fig.15: einen Teilschnitt eines Kanalabschnitts zur Durchleitung der Atemluft mit einem Sperrgllied gemäß Fig. 13 im Schnitt, vergrößert und
- Fig.16: ein Teilstück eines Rüssels abgewandelter Ausgestaltung in Seitenansicht.

Die Fig. 1 und 2 zeigen eine Vorrichtung mit einem halbkugelförmigen Gehäuse 1, das im Endflächenbereich 2 durch eine Trennwand 3 verschlossen ist. Der Endflächenbereich 2 trägt einen Aufsatz 3', der rohrförmige Kanäle 4,5 und 6 aufnimmt. Der Kanal 4 dient der Verbindung der Vorrichtung mit einem Luftröhrenkatheter (nicht gezeigt), während der Kanal 5 die Durchleitung von Atemluft ermöglicht. Kanal 6 ist für die Abführung von Sputum vorgesehen. Mit dem Kanal 6 ist ein abbiegbarer rohrförmiger Rüssel 7 verbunden, der sich quer zur Mittellängsachse des Kanals 6 erstreckt und aus einem hoch flexiblen oder federnd elastischen Werkstoff gebildet ist. Der Rüssel 7 kann aus der voll gezeichneten Stellung der Fig. 1 in der Ebene derselben und in beliebigen seitlichen Stellungen abbiegbar sein. Bei Eintritt von Sputum in das Gehäuse 1, etwa bei Hustenstößen fließt das Sputum zunächst in die untere Kugelzone 1' und von dort durch Einwirkung der Atemluft oder des Hustendrucks in den Rüssel 7 und weiter in den Kanal 6 zur Abführung nach außen.

In Fig. 3 ist der Rüssel 7 an seinem freien Ende außen mit einem ringförmigen Gewichtskörper 33 ausgestattet, der dafür sorgt, daß der Rüssel 7 stets die jeweils untere Zone 1' des Gehäuses 1 kontaktiert. An seinem dem Kanal 6 zugewandten Ende weist der Rüssel 7 eine Rücklaufsperre 8 für Sputum auf. Die Rücklaufsperre 8 ist in den Fig. 9 und 10 durch einen Ringkörper 9 mit einer Anzahl aufklappbarer Segmente 9' gebildet, die unter der Einwirkung von Atemdruck durch Sputum aufgebogen und bei Beendigung des auf das Sputum einwirkenden Atemdrucks durch Rückbiegung die Rücklaufsperre 8 schließen. Der Kanal 5 trägt außen einen Luftfilter 10, z.B. mit einem Filterkörper 11 aus Schaumstoff od.dgl., der in der Ausführung gemäß der Fig. 11 im Bereich der Offenseite durch ein Gitter 10' verschlossen und desweiteren mit einer Heizeinrichtung ausgestattet ist. Die Heizeinrichtung ist einfach durch einen Widerstandsdraht 12 gebildet, der bevorzugt an eine Niederspannungsquelle anlegbar ist.

In Fig. 4 ist eine Vorrichtung mit einem kugelförmigen Gehäuse 1 dargestellt, das wiederum Kanäle 4 bis 6 aufnimmt, bei dem der Sputumkanal 6 zusätzlich mit einer Pumpe 13 ausgerüstet ist. Die Pumpe 13 ist beim Ausführungsbeispiel als Kolbenpumpe ausgebildet, die das Sputum über den Rüssel 7 ansaugt und in den Kanal 6 fördert. Der Kolben 13' der Pumpe 13 ist mit einem Plattenkörper 14 verbunden, der an einer gehäusefesten gewellten Manschette 15 aus federnd elastischem Werkstoff angreift.

Bei Hustenstößen gelangt Sputum aus der jeweils unteren Kugelzone 1' des Gehäuses 1 in den Rüssel 7, über das Einlaßventil 13"" in den Pumpenzylinder 13" und über das Auslaßventil 13"' in den Kanal 6. Hierbei wird der Plattenkörper 14 zunächst mitsamt dem Kolben 13' entgegen der Vorspannung der Manschette 15 aufwärts (Fig.5) und bei Beendigung der Hustenstöße od.dgl. durch die Vorspannung der Manschette 15 zurück in die Ruhestellung abwärts bewegt.

Beim Ausführungsbeispiel der Fig. 6 ist in Abweichung das Gehäuse 1 der Vorrichtung durch zwei sich einander mit Abstand umfassende Kugelkörper 16, 17 gebildet, wobei der Zwischenraum 18 zwischen den beiden Kugelkörpern 16, 17 einen Filterwerkstoff 19 aufnimmt, der durch eine gelochte Folie 20 innen begrenzt ist. Die Folie 20 ist unter Bildung eines Spaltenraums 24 im Abstand des inneren Kugelkörpers 16 durch Abstandshalter 21 abgestützt. Der Filterwerkstoff 19 ist mit einer Aussparung 22 versehen, an die der Kanal 5 für die Durchleitung der Atemluft anliegt. Während beim Ausführungsbeispiel der innere Kugelkörper 16 geschlossen ausgebildet ist, ist der äußere Kugelkörper 17 mit Luftzuführungsöffnungen 23 versehen. die Atemluft kann so über die Luftzuführungsöffnungen 23 den Filterwerkstoff 19 durchsetzen und in den Spaltenraum 24 eintreten und weiter in die Aussparung 22 gelangen. Der Kanal 6 für die Abführung des Sputums ist auch bei dieser Ausführung mit einem Rüssel 7 ausgestattet, über den das Sputum aus dem inneren Kugelkörper 16 in den Kanal 6 gelangt.

Es ist denkbar, den Rüssel 7 mit dem freien Ende an die Innenwand des Gehäuses 1 anliegend auszubilden, wobei zur Einleitung von Sputum in den Rüssel 7 Durchtrittsöffnungen 25 oder Aussparungen am freien Ende desselben vorzusehen sind. (Fig. 16)

In Fig. 12 ist auf dem Sputumkanal 6 eine topfförmige Hülse 26 drehbar aufgesetzt, in die der Rüssel 7 mit seinem kanalnahen Ende einmündet. Der Rüssel 7 ist vermittels der Hülse 26 um 360 Grad auf dem Sputumkanal 6 verschwenkbar und zusätzlich durch seine Flexiblität zu beiden Seiten aus der gestreckten Stellung abbiegbar. Auf diese Weise ist sichergestellt, daß die gesamte Innenwandung des jeweiligen Gehäuses 1, 16, 17 abtastbar und die Abführung des Sputums gewährleistet ist.

Die Fig. 7 und 8 zeigen einen Kanal 5 für die Durchleitung von Atemluft, der durch einen Schieber 27 oder Klappe über einen Teilquerschnitt versperrt ist. Durch den Sperrschieber 27 wird im Gehäuse 1 bei Hustenstößen eine Druckerhöhung erreicht, die zum sicheren Einleiten von Sputum in den Rüssel 7 und Transportieren des Sputums in den Kanal 6 sorgt. Der als Sperre wirkende Schieber 27 ist durch Atemluft zur Freigabe des Kanals 5 verschiebbar, wozu die Atemluft auf eine Membrane 28 im Zylinder 29 auftrifft und über ein Gestänge 30 den Schieber 27 anhebt. Als Sperre kann eine ebene geschlossene oder gewölbte Platte (Fig. 8) oder ein Siebkörper 31 mit Rahmen 31' dienen.

In den Fig. 13 und 14 ist im Kanal 5 eine als Staukörper wirkende Zunge 32 aus einem dünnwandigen flexiblen Werkstoff mit einem Ende festgelegt, der unter dem Einfluß von Atemluft in die eine oder andere Bewegungsrichtung abkippbar ist. Der Staukörper 32 wirkt als Sperrglied und ist im Querschnitt bogenförmig gestaltet, wodurch der Staukörper in einer Bewegungsrichtung der Atemluft mit geringerem Druck als in der anderen Bewegungsrichtung der Atemluft abkippbar ist. Bei Fehlen von Atemluftströmung richtet sich der Staukörper 32 unter dem Einfluß der Werkstoffspannungen selbsttätig in die in der Fig. 13 gezeigten aufrechten Stellung auf.

## Patentansprüche

1. Vorrichtung zum Trennen von Gas- und Flüssigkeitsströmen, an Luftröhrenkathetern von Patienten mit einem Kanäle (4,5,6) für die Zu- und Abführung von Atemluft und der Abführung von Sputum aufweisenden Gehäuse (1), **gekennzeichnet durch** die Anordnung eines mindestens kugelabschnittsförmigen geschlossenen Gehäuses (1) mit im Endflächenbereich (2) des Gehäuses (1) im Abstand voneinander angeordneten rohrförmigen Kanälen (4, 5) für eine Verbindung mit dem Luftröhrenkatheter und zur Durchleitung von Atemluft sowie eines rohrförmigen Kanals (6) zur Abführung von Sputum nach außen, dessen inneres Stirnende verschlossen ist und an dessen Kanal-innenraum ein **durch** Schwerkraft selbständig abbiegbarer und/öder abschwenkbarer rohrförmiger Rüssel (7) aus einem hoch flexiblen oder federnd elastischen Werkstoff anliegt, der quer zum Kanal für die Abführung von Sputum (6) sich erstreckend ausgebildet ist und mit seinem freien Ende im Abstand bis annähernd der Innenwand des Gehäuses (1) reicht.

2. Vorrichtung nach Anspruch 1, **dadurch gekennzeichnet, daß** das Gehäuse (1) als Halbkugel ausgebildet ist und die Kanäle (4,5,6) im Endflächenbereich (2) des Gehäuses (1) angeordnet sind.

3. Vorrichtung nach Anspruch 1, **dadurch gekennzeichnet, daß** das Gehäuse (1) als Vollkugel ausgebildet ist und die Kanäle (4,5,6) in einer durch den Bereich des Kugelmittelpunkts geführten Ebene angeordnet sind.

4. Vorrichtung nach Anspruch 1, **dadurch gekennzeichnet, daß** das Gehäuse (1) kugelabschnittsförmig, insbesondere halbkugelförmig ausgebildet ist und getrennt unabhängig auf der Endfläche einen kammerförmigen Aufsatz (3') trägt, der den Kanal (4) für die Verbindung zu dem Luftröhrenkatheter und den Kanal (5) für die Durchleitung der Atemluft sowie den Kanal (6) für die Abführung des Sputums aufnimmt und wobei der Rüssel (7) durch eine Lochung der Trennwand (3) von Aufsatz (3') und Gehäuse (1) in das Gehäuse (1) einragt und über seine Länge abbieg- oder abschwenkbar im Abstand bis annähernd der Innenwand des Gehäuses (1) geführt ist.

5. Vorrichtung nach Anspruch 1, **dadurch gekennzeichnet, daß** der Rüssel (7) frei abbiegbar ausgebildet ist und mit dem kanalseitigen Ende auf dem Kanal (6) für die Abführung des Sputums um die Längsachse des Kanals (6) frei drehbar dicht ausgebildet ist.

6. Vorrichtung nach Anspruch 5, **dadurch gekennzeichnet, daß** der Rüssel (7) mit seinem kanalseitigen Ende in eine topfförmige Hülse (26) einmündet und vermittels der Hülse auf dem Kanal (6) für die Abführung von Sputum um die Mittellängsachse desselben frei drehbar ist.

7. Vorrichtung nach Anspruch 1, **dadurch gekennzeichnet**, daB die Kanäle (6, 6) für die Abführung des Sputums und für die Durchleitung der Atemluft gemeinsam relativ zum Gehause (1) verschwenk- oder verdrehbar ausgebildet sind.

8. Vorrichtung nach Anspruch 1, 2, 5 und 6, **dadurch gekennzeichnet, daß** der Rüssel (7) mit dem freien Ende an der Innenwand des Gehäuses (1) anliegt und an dem der Innenwand zugeordneten Ende Durchführungsöffnungen (25) od.dgl. für die Einleitung von Sputum in den Rüssel (7) aufweist.

9. Vorrichtung nach Anspruch 5 und 6, **dadurch gekennzeichnet, daß** der Rüssel (7) am freien Ende außen einen Körper, z.B. Ringkörper (33), mit großem spezifischen Gewicht trägt und daß der Rüssel (7) gemeinsam durch den Gewichtskörper (33) im Gehäuse (1) mit radialer Ausrichtung zum Kanal (6) für die Sputum-Abführung bring- und haltbar ist.

10. Vorrichtung nach Anspruch 1, **dadurch gekennzeichnet, daß** am Kanal (6) für die Abführung des Sputums eine Pumpe (13) mit ihrem Auslaß (13''') anliegt, deren Eingang (13'''') mit dem Rüssel (7) verbunden ist.

11. Vorrichtung nach Anspruch 10, **dadurch gekennzeichnet, daß** die Pumpe (13) als Kolben- oder Membranpumpe ausgebildet ist und daß der Kolben (13') oder die Membrane der Pumpe (13) durch Atemdruck aus der Ruhestellung in die Förderstellung für Sputum bringbar und bei Fehlen von Atemdruck durch Feder- und/oder Schwerkraft in die Ruhestellung selbsttätig rückführbar sind.

12. Vorrichtung nach Anspruch 11, **dadurch gekennzeichnet, daß** die Federkraft durch einen verformbaren Plattenkörper aus einem federnd elastischen Werkstoff bildbar ist, der äm Gehäuse (1) oder dem Kanal (6) für die von Sputum fixiert und durch ein Betätigungsglied mit dem Kolben oder der Membrane der Pumpe fest verbunden ist.

13. Vorrichtung nach Anspruch 11, **dadurch gekennzeichnet, daß** die Federkraft durch einen an einer gewellten Manschette (15) aus federnd elastischem Werkstoff angeordneten starren Plattenkörper (14) aufbringbar ist und der Plattenkörper (14) durch ein Betätigungsglied mit dem Kolben (13') oder der Membrane der Pumpe (13) verbunden ist.

14. Vorrichtung nach Anspruch 1, **dadurch gekennzeichnet, daß** der Kanal (6) für die Abführung des Sputums zur Verhinderung unbeabsichtigter Rückflüsse von Sputum eine Rückflußsperre mit in Rückflußrichtung (8) ausgerichteter Sperrwirkung aufweist.

15. Vorrichtung nach Anspruch 1, **dadurch gekennzeichnet, daß** der Kanal (5) für die Zuleitung der Atemluft in Ruhestellung über einen Teilquerschnitt durch einen Schieber (27) oder eine Klappe verdeckt ausgebildet ist und daß der Schieber oder die Klappe durch abfließende Atemluft und/oder Hustenstöße zur Freigabe eines größeren oder des ganzen Querschnitts des Kanals (5) für die Atemluft selbsttätig verschieb- oder verschwenkbar ist oder in Einatmungsrichtung öffnet.

16. Vorrichtung nach Anspruch 1, **dadurch gekennzeichnet, daß** der Kanal (5) für die Zuleitung der Atemluft in Ruhestellung über einen Teilquerschnitt durch eine Zunge (32) aus flexiblen oder federnd elastischen Werkstoff versperrt ausgebildet ist, die Zunge (32) durch zufließende Atemluft zur Vergrößerung des Durchtrittsquerschnitts von Atemluft umklappbar ist und durch abfließende Atemluft entgegen dem Verformungswiderstand rückschwenkbar ist.

17. Vorrichtung nach.Anspruch 1, 14, 15, 16 **dadurch gekennzeichnet, daß** der rohrförmige Kanal (5) für die Durchleitung der Atemluft eingangsseitig außen einen Luftfilter (10) trägt.

18. Vorrichtung nach Anspruch 17, **dadurch gekennzeichnet, daß** der Luftfilter (10) zur Vorwärmung zuzuführender Atemluft eine wahlweise manuell periodisch oder über einen Sensor selbsttätig zuschaltbare elektrische Heizeinrichtung aufweist.

19. Vorrichtung nach Antpruch 17 und 18, **dadurch gekennzeichnet, daß** der Filterkörper des Luftfilters (10) und/oder die Atemzuluft über öffnungen im Filtergehäuse mit flüssigen Medien befeuchtbar ist.

20. Vorrichtung nach Anspruch 1 bis 3, **dadurch gekennzeichnet, daß** ein flüssiges Medium als Spülmittel in den Gehäuseinnenraum, z.B. durch eine Pumpe od.dgl., einbringbar ist.

21. Vorrichtung nach Anspruch 1, **dadurch gekennzeichnet, daß** das Gehäuse (1) durch je einen im Abstand einander zentrisch umfassenden Außen-(17) und Innenkörper (16), gebildet ist daß der Zwischenraum (18) der beiden Körper (16, 17) einen schaum- oder schwammförmigen Filterwerkstoff (19) aufnimmt, der mit Abstand zum Innenkörper (16) durch eine Folie (20) begrenzt ist, wobei die Folie (20) durch Abstandshalter (21) an dem Innenkörper (16) zur Bildung eines umlaufenden Spaltenraumes (24) abgestützt und mit einer Anzahl Luftdurchtrittsöffnungen versehen ist, der Filterwerkstoff (19) über eine Teillänge ausgespart ist und daß an der Aussparung (22) der Kanal (5) für die Durchleitung der Atemluft anliegt und der Außenkörper (17) mit Durchflußöffnungen (23) für die Atemluft versehen ist.

22. Vorrichtung zum Trennen von Gas- und Flüssigkeitsströmen, insbesondere an Luftröhrenkathetern von Patienten mit einem Kanäle für die Zu- und Abführung von Atemluft und der Abführung von Sputum aufweisenden Gehäuse, **gekennzeichnet durch** ein **durch** einen an den stirnseitigen Enden und den Umfangsflächen geschlossenen Zylinder gebildetes Gehäuse (1) mit im Endflächenbereich (2) des Gehäuses (1) im Abstand voneinander angeordneten rohrförmigen Kanälen (4, 5) für eine Verbindung mit dem Luftröhrenkatheter und zur Durchleitung von Atemluft sowie eines rohrförmigen Kanals (6) zur Abführung von Sputum nach außen, dessen inneres Stirnende verschlossen ist und an dessen Kanal-innenraum ein **durch** Schwerkraft selbständig abbiegbarer und/oder abschwenkbarer rohrförmiger Rüssel (7) aus einem hoch flexiblen oder federnd elastischen Werkstoff anliegt, der quer zum Kanal (6) für die Abführung von Sputum sich erstreckend ausgebildet ist und mit seinem freien Ende im Abstand bis annähernd der Innenwand des Gehäuses (1) reicht, wobei
- der Rüssel (7) frei abbiegbar ausgebildet ist und mit dem kanalseitigen Ende auf dem Kanal (6) für die Abführung des Sputums um die Längsachse des Kanals (6) frei drehbar dicht ausgebildet ist,
- der Rüssel (7) mit seinem kanalseitigen Ende in eine topfförmige Hülse (26) einmündet und vermittels der Hülse auf dem Kanal (6) für die Abführung von Sputum um die Mittellängsachse desselben frei drehbar ist,
- die Kanäle (6, 5) für die Abführung des Sputums und für die Durchleitung der Atemluft gemeinsam relativ zum Gehäuse (1) verschwenk- oder verdrehbar ausgebildet sind,
- der Rüssel (7) mit dem freien Ende an der Innenwand des Gehäuses (1) anliegt und an dem der Innenwand zugeordneten Ende Durchführungsöffnungen (25) od.dgl. für die Einleitung von Sputum in den Rüssel (7) aufweist,
- der Rüssel (7) am freien Ende außen einen Körper, z.B. Ringkörper (33) mit großem spezifischen Gewicht trägt und daß der Rüssel (7) gemeinsam **durch** den Gewichtskörper (33) im Gehäuse (1) mit radialer Ausrichtung zum Kanal (6) für die Sputum-Abführung bring- und haltbar ist,
- am Kanal (6) für die Abführung des Sputums eine Pumpe (13) mit ihrem Auslaß (13'") anliegt, deren Eingang (13'''') mit dem Rüssel (7) verbunden ist,
- die Pumpe (13) als Kolben- oder Membranpumpe ausgebildet ist und daß der Kolben (13') oder die Membrane der Pumpe (13) **durch** Atemdruck aus der Ruhestellung in die Förderstellung für Sputum bringbar und bei Fehlen von Atemdruck **durch** Feder- und/oder Schwerkraft in die Ruhestellung selbsttätig rückführbar sind,
- die Federkraft **durch** einen verformbaren Plattenkörper aus einem federnd elastischen Werkstoff bildbar ist, der am Gehäuse (1) oder dem Kanal (6) für die von Sputum fixiert und **durch** ein Betätigungsglied mit dem Kolben oder der Membrane der Pumpe fest verbunden ist,
- die Federkraft **durch** einen an einer gewellten Manschette (15) aus federnd elastischem Werkstoff angeordneten starren Plattenkörper (14) aufbringbar ist und der Plattenkörper (14) **durch** ein Betätigungsglied mit dem Kolben (13') oder der Membrane der Pumpe (13) verbunden ist und
- der Kanal (6) für die Abführung des Sputums zur Verhinderung unbeabsichtigter Rückflüsse von Sputum eine Rückflußsperre mit in Rückflußrichtung (8) ausgerichteter Sperrwirkung aufweist.

## Claims

1. A device for separating gas and liquid streams, in tracheal catheters of patients, with a housing (1) having channels (4. 5, 6) for the delivery and removal of respiratory air and for the removal of sputum, **characterized by** the arrangement of an at least spherical segment-shaped closed housing (1) with tubular channels (4, 5) which are arranged at a distance from one another in the area of the end face (2) of the housing (1) and each lead to a connection of the channels to the tracheal catheter and are for the passage of respiratory air, and of a tubular channel (6), for removal of sputum to the outside, whose inner front end is sealed and which has, bearing on its channel interior, a tubular suction nozzle (7) made of a highly flexible or resiliently elastic material, which suction nozzle (7) can be bent and/or swiveled aside automatically by the force of gravity, is designed extending transversely with respect to the channel (6) for the deduction of sputum and, with its free end, reaches approximately of the inner wall of the housing (1).

2. The device as claimed in claim 1, **characterized in that** the housing (1) is designed as a hemisphere and the channels (4, 5, 6) are arranged in the area of the end face (2) of the housing (1) .

3. The device as claimed in claim 1, **characterized in that** the housing (1) is designed as a full sphere and the channels (4, 5, 6) are arranged in a plane passing through the area of the midpoint of the sphere.

4. The device as claimed in claim 1, **characterized in that** the housing (1) is spherical segment-shaped, in particular hemispherical, and has, separately and independently, on the end face, a chamber-like attachment (3') which receives the channel (4) for the connection to the tracheal catheter and the channel (5) for passage of respiratory air and the channel (6) for removal of sputum, and the suction nozzle (7) extends into the housing (1) through a hole in the dividing wall (3) between attachment (3') and housing (1), and, being able to be bent or swiveled aside along its length, is guided approximately of the inner wall of the housing (1) .

5. The device as claimed in claim 1, **characterized in that** the suction nozzle (7) is designed to be freely bendable and, with the channel-side end arranged tightly on the channel (6) for removal of sputum, is designed to be freely rotatable about the longitudinal axis of the channel (6).

6. The device as claimed in claim 5, **characterized in that** the suction nozzle (7) opens with its channel-side end into a pot-shaped sleeve (26) and, by way of the sleeve, is freely rotatable on the channel (6) for the deduction of sputum about the central longitudinal axis thereof.

7. The device as claimed in claim 1, **characterized in that** the channels (6, 5) for the removal of sputum and the passage of respiratory air are designed to be jointly pivotable or rotatable relative to the housing (1).

8. The device as claimed in claims 1, 2, 5 and 6, **characterized in that** the suction nozzle (7) bears with its free end on the inner wall of the housing (1) and, at the end facing the inner wall, has through-openings (25) or the like for introduction of sputum into the suction nozzle (7).

9. The device as claimed in claims 5 and 6, **characterized in that**, on the outside of the free end, the suction nozzle (7) supports a body, for example an annular body (33), of high specific weight, and **in that**, by means of this weight body (33), the suction nozzle (7) jointly with sleeve (26), can be brought to and held in the housing (1) at a radial orientation with respect to the channel (6) for sputum removal.

10. The device as claimed in claim 1, **characterized in that** a pump (13) bears with its outlet (13''') on the channel (6) for removal of sputum, its inlet (13'''') being connected to the suction nozzle (7).

11. The device as claimed in claim 10, **characterized in that** the pump (13) is designed as piston pump or diaphragm pump, and **in that** the piston (13') or the diaphragm of the pump (13) can be brought, by respiratory pressure, from the rest position to the conveying position for sputum and, in the absence of respiratory pressure, can be returned automatically to the rest position by resiliency and/or gravity.

12. The device as claimed in claim 11, **characterized in that** the resiliency can be formed by a deformable plate body made of a resiliently elastic material, which plate body is fixed on the housing (1) or the channel (6) for of sputum and is securely connected to the piston or the diaphragm of the pump by an actuating member.

13. The device as claimed in claim 11, **characterized in that** the resiliency can be applied by a rigid plate body (14) arranged on a corrugated cuff (15) made of resiliently elastic material, and the plate body (14) is connected to the piston (13') or the diaphragm of the pump (13) by an actuating member.

14. The device as claimed in claim 1, **characterized in that** the channel (6) for removal of sputum has, in order to avoid inadvertent reflux of sputum, a reflux barrier, with a barrier action oriented in the reflux direction (8).

15. The device as claimed in claim 1, **characterized in that**, in the rest position, the channel (5) for delivery of respiratory air is covered over part of its cross section by a slide (27) or a flap, and **in that** the slide or the flap can be automatically displaced or pivoted, or opened in the inhalation direction, by outflowing respiratory air and/or coughs so as to free a greater cross section or the whole cross section of the channel (5) for respiratory air.

16. The device as claimed in claim 1, **characterized in that**, in the rest position, the channel (5) for delivery of respiratory air is covered over part of its cross section by a tongue (32) made of flexible or resiliently elastic material, the tongue (32) can be tilted by incoming respiratory air so as to increase the cross section of the passage of respiratory air and can be swiveled back by outflowing respiratory air counter to the deformation resistance.

17. The device as claimed in claim 1, 14, 15, 16, **characterized in that** the inlet end of the tubular channel (5) for the passage of respiratory air has an air filter (10) on the outside.

18. The device as claimed in claim 17, **characterized in that** the air filter (10), for the purpose of preheating incoming respiratory air, has an electrical heating arrangement, for example a heating coil (12) or the like, which can either be switched on manually at periods or can be switched on automatically via a sensor.

19. The device as claimed in claims 17 and 18, **characterized in that** the filter body of the air filter (10) and/or the incoming respiratory air can be moistened with liquid media via openings in the filter housing.

20. The device as claimed in claims 1 through 3, **characterized in that** a liquid medium acting as flushing agent can be introduced into the housing interior, for example by a pump or the like.

21. The device as claimed in claim 1, **characterized in that** the housing (1) is formed by an outer body (17) and an inner body (16), one centrically enclosing the other at a distance, **in that** the interspace (18) between the two bodies (16, 17) receives a foam-like or sponge-like filter material (19) which is delimited at a distance from the inner body (16) by a foil (20), said foil (20) being supported by spacers (21) on the inner body (16) in order to form a peripheral gap (24) and being provided with a number of air through-openings, the filter material (19) being left open along part of its length, and **in that** the channel (5) for the passage of respiratory air bears on the recess (22), and the outer body (17) is provided with through-flow openings (23) for the respiratory air.

22. A device for separating gas and liquid streams, in tracheal catheters of patients, with a housing (1) having channels (4, 5, 6) for the delivery and removal of respiratory air and for the removal of sputum, **characterized by** a housing formed by a cylinder closed at the end faces and on the circumferential surfaces with tubular channels (4, 5) which are arranged at a distance from one another in the area of the end face (2) of the housing (1) and each lead to a connection of the channels to the tracheal catheter and are for the passage of respiratory air, and of a tubular channel (6), for removal of sputum to the outside, whose inner front end is sealed and which has, bearing on its channel interior, a tubular suction nozzle (7) made of a highly flexible or resiliently elastic material, which suction nozzle (7) can be bent and/or swiveled aside automatically by the force of gravity, is designed extending transversely with respect to the channel (6) for the deduction of sputum and, with its free end, reaches approximately of the inner wall of the housing (1) whereby
- the suction nozzle (7) is designed to be freely bendable and, with the channel-side end arranged tightly on the channel (6) for removal of sputum, is designed to be freely rotatable about the longitudinal axis of the channel (6),
- the suction nozzle (7) opens with its channel-side end into a pot-shaped sleeve (26) and, by way of the sleeve, is freely rotatable on the channel (6) for the deduction of sputum about the central longitudinal axis thereof,
- the channels (6, 5) for the removal of sputum and the passage of respiratory air are designed to be jointly pivotable or rotatable relative to the housing (1),
- the suction nozzle (7) bears with its free end on the inner wall of the housing (1) and, at the end facing the inner wall, has through-openings (25) or the like for introduction of sputum into the suction nozzle (7),
- on the outside of the free end, the suction nozzle (7) supports a body, for example an annular body (33), of high specific weight, and in that, by means of this weight body (33), the suction nozzle (7) jointly with sleeve (26), can be brought to and held in the housing (1) at a radial orientation with respect to the channel (6) for sputum removal,
- a pump (13) bears with its outlet (13"') on the channel (6) for removal of sputum, its inlet (13"") being connected to the suction nozzle (7),
- a pump (13) is designed as piston pump or diaphragm pump, and in that the piston (13') or the diaphragm of the pump (13) can be brought, by respiratory pressure, from the rest position to the conveying position for sputum and, in the absence of respiratory pressure, can be returned automatically to the rest position by resiliency and/or gravity,
- the resiliency can be formed by a deformable plate body made of a resiliently elastic material, which plate body is fixed on the housing (1) or the channel (6) for of sputum and is securely connected to the piston or the diaphragm of the pump by an actuating member,
- the resiliency can be applied by a rigid plate body (14) arranged on a corrugated cuff (15) made of resiliently elastic material, and the plate body (14) is connected to the piston (13') or the diaphragm of the pump (13) by an actuating member,
- the channel (6) for removal of sputum has, in order to avoid inadvertent reflux of sputum, a reflux barrier, with a barrier action oriented in the reflux direction (8).

## Revendications

1. Dispositif pour séparer des flux de gaz et de liquide, dans des sondes d'aspiration trachéales de patients, comprenant une enveloppe (1) présentant des canaux (4, 5, 6) pour l'admission et le refoulement d'air respiré ainsi que pour l'évacuation des expectorations, **caractérisé par** la disposition d'une enveloppe fermée (1) ayant la forme d'au moins une calotte sphérique, en combinaison avec des canaux (4, 5), de forme tubulaire, disposés, dans la zone de surface terminale (2) de l'enveloppe (1), en espacement l'un de l'autre, chacun desdits canaux étant en communication avec la sonde d'aspiration trachéale et susceptible de véhiculer l'air respiré, ainsi qu'en communication avec un canal (6), de forme tubulaire, pour l'évacuation des expectorations vers l'extérieur, l'extrémité interne frontale dudit canal étant fermée tandis qu'avec son espace intérieur de canal vient en ajustement d'appui une trompe (7), de forme tubulaire, susceptible de pivoter et/ou de se déformer par elle-même grâce à la force de gravitation, réalisée en un matériau de flexibilité élevée ou ayant l'élasticité d'un ressort, ladite trompe (7) étant de conformation telle qu'elle s'étend de façon transversale par rapport au canal (6) pour l'évacuation des expectorations et telle que son extrémité libre est séparée mais jusqu'à s'approcher de la paroi interne de l'enveloppe (1).

2. Dispositif selon la revendication 1, **caractérisé en ce que** l'enveloppe (1) est une demi-sphêre et **en ce que** les canaux (4, 5, 6) sont disposés dans la zone de surface terminale (2) de l'enveloppe (1).

3. Dispositif selon la revendication 1, **caractérisé en ce que** l'enveloppe (1) est une sphère complète et **en ce que** les canaux (4, 5, 6) sont disposés dans un plan traversant la zone centrale de la sphère.

4. Dispositif selon la revendication 1, **caractérisé en ce que** l'enveloppe (1) présente une forme de calotte sphérique, notamment en forme de demi-sphère, et porte, séparément et indépendamment sur sa surface terminale, un élément supérieur (3') en forme de chambre, qui reçoit le canal (4) pour la communication avec la sonde d'aspiration trachéale et le canal (5) pour véhiculer l'air respiré, ainsi que le canal (6) pour l'évacuation des expectorations, et **en ce que** la trompe (7) s'engage, par le biais d'un évidemment de la paroi (3) assurant la séparation de l'élément supérieur (3') et de l'enveloppe (1), dans ladite enveloppe (1) et est amenée, du fait de sa longueur et de façon déformable ou pivotable, jusqu'au voisinage immédiat de la paroi interne de l'enveloppe (1).

5. Dispositif selon la revendication 1, **caractérisé en ce que** la trompe (7) est librement déformable en courbure et **en ce qu'**elle est conformée en rotation libre et étanche autour de l'axe longitudinal du canal (6), avec son extrémité côté canal engagée sur le canal (6) pour l'évacuation des expectorations.

6. Dispositif selon la revendication 5, **caractérisé en ce que** la trompe (7) débouche par son extrémité côté canal dans une douille en forme de pot (26) et est libre en rotation, au moyen de la douille engagée sur le canal (6) pour l'évacuation des expectorations, autour de l'axe longitudinal médian de celui-ci.

7. Dispositif selon la revendication 1, **caractérisé en ce que** les canaux (6, 5) pour l'évacuation des expectorations et pour véhiculer l'air respiré sont conjointement conformés, par rapport à l'enveloppe (1), en étant susceptibles de pivotement ou de rotation.

8. Dispositif selon l'une des revendications 1, 2, 5, 6, **caractérisé en ce que** la trompe (7) repose, par son extrémité libre, sur la paroi interne de l'enveloppe (1) et présente, à son extrémité dirigée vers ladite paroi interne, des ouvertures de passage (25) ou analogues pour l'admission des expectorations dans la trompe (7).

9. Dispositif selon la revendication 5 ou 6,
**caractérisé en ce que** la trompe (7) porte à l'extérieur de son extrémité libre un corps, par exemple un corps annulaire (33) de poids spécifique élevé et **en ce que** la trompe (7), conjointement avec le corps de poids (33) est susceptible d'être amenée et maintenue dans l'enveloppe (1), en ajustement radial par rapport au canal (6) pour l'évacuation des expectorations.

10. Dispositif selon la revendication 1, **caractérisé en ce que** sur le canal (6) pour l'évacuation des expectorations est ajustée une pompe (13) par son refoulement (13 "'), tandis que l'admission (13'''') de ladite pompe est en communication avec la trompe (7).

11. Dispositif selon la revendication 10, **caractérisé en ce que** la pompe (13) est une pompe à piston ou à membrane et **en ce que** le piston (13') ou la membrane de la pompe (13) sont susceptibles d'être amenés, par le biais de la pression de respiration, depuis la position de repos jusqu'en position de pompage des expectorations et sont susceptibles d'être ramenés automatiquement en position de repos par le biais d'une force élastique et/ou de gravitation en cas d'absence d'air respiré.

12. Dispositif selon la revendication 11, **caractérisé en ce que** la force élastique est susceptible d'être engendrée par un corps formant plaque, déformable et en un matériau ayant l'élasticité d'un ressort, ledit corps formant plaque étant fixé à l'enveloppe (1) ou au canal (6) pour l'évacuation des expectorations et étant solidaire par le biais d'un organe d'actionnement avec le piston ou la membrane de la pompe.

13. Dispositif selon la revendication 11, **caractérisé en ce que** la force élastique est susceptible d'être appliquée par le biais d'un corps rigide formant plaque (14) disposé le long d'un manchon à profil ondulé (15), en un matériau ayant l'élasticité d'un ressort, et **en ce que** le corps formant plaque (14) est relié, par le biais d'un organe d'actionnement, au piston (13') ou à la membrane de la pompe (13).

14. Dispositif selon la revendication 1, **caractérisé en ce que** le canal (6) pour l'évacuation des expectorations présente, afin d'empêcher des reflux indésirables d'expectoration, une barrière de reflux, avec une action de barrière ajustable dans le sens du reflux (8).

15. Dispositif selon la revendication 1, **caractérisé en ce que** le canal (5) pour l'admission de l'air respiré est conformé en position de repos sur une section transversale partielle, par un tiroir (27) ou par un clapet et **en ce que** le tiroir ou le clapet s'ouvre automatiquement soit par coulissement, soit par pivotement soit dans le sens de l'air inspiré, par le biais de l'écoulement de l'air respiré et/ou des quintes de toux, en vue de débloquer une assez grande partie ou la totalité de la section transversale du canal (5) pour l'air respiré.

16. Dispositif selon la revendication 1, **caractérisé en ce que** le canal (5) pour véhiculer l'air respiré est conformé de façon à être obturé en position de repos sur une section transversale partielle par une languette (32) en un matériau flexible ou ayant l'élasticité d'un ressort, et **en ce que** la languette (32) est susceptible d'un mouvement de rabattement par le biais de l'admission d'air respiré en vue d'augmenter la section transversale de passage de l'air respiré et est susceptible du mouvement inverse de retour en position, par le biais de l'écoulement de l'air respiré antagoniste à la résistance à la déformation.

17. Dispositif selon l'une des revendications 1, 14, 15, 16, **caractérisé en ce que** le canal de forme tubulaire (5) pour véhiculer l'air respiré comporte, à l'extérieur côté entrée, un filtre à air (10).

18. Dispositif selon la revendication 17, **caractérisé en ce que** le filtre à air (10) présente, pour le préchauffage de l'air inspiré, un dispositif de chauffage électrique, par exemple une bobine de chauffage (12) en analogue, susceptible d'être alimenté au choix de façon manuelle et périodique ou automatiquement par le biais d'un capteur.

19. Dispositif selon la revendication 17 et 18,
**caractérisé en ce que** l'élément filtrant du filtre à air (10) et/ou l'air inspiré est susceptible d'être humidifié par des milieux liquides, par le biais d'ouvertures pratiquées dans le boîtier du filtre.

20. Dispositif selon l'une des revendications 1 à 3, **caractérisé en ce qu'**un milieu liquide est susceptible d'être amené en tant qu'agent de pulvérisation dans l'espace interne de l'enveloppe, par exemple par une pompe ou analogue.

21. Dispositif selon la revendication 1, **caractérisé en ce que** l'enveloppe (1) est constituée d'un corps externe (17) entourant un corps interne (16) selon une disposition mutuelle concentrique espacée, **en ce que** l'espace intermédiaire (18) entre les deux corps (16, 17) reçoit un matériau filtrant (19) sous forme de mousse ou d'éponge, qui est séparé du corps interne (16) par une pellicule (20), ladite pellicule (20) étant en appui, par le biais d'écarteurs (21), sur le corps interne (16) pour la formation d'un espace continu de partition (24) et étant munie d'une pluralité d'ouvertures pour le passage de l'air, tandis que le matériau filtrant (19) est absent sur une longueur partielle, et **en ce que** le long de l'évidement (22) se situe le canal (5) pour véhiculer l'air respiré, tandis que le corps externe (17) est muni d'ouvertures (23) pour l'écoulement de l'air respiré.

22. Dispositif pour séparer des flux de gaz et de liquide, dans des sondes d'aspiration trachéales de patients, comprenant une enveloppe (1) présentant des canaux (4, 5, 6) pour l'admission et le refoulement d'air respiré ainsi que pour l'évacuation des expectorations, **caractérisé en ce que** l'enveloppe (1) est constituée d'un cylindre (29) fermé à ses extrémités frontales et sur sa surface périphérique en combinaison avec des canaux (4, 5), de forme tubulaire, disposés, dans la zone de surface terminale (2) de l'enveloppe (1), en espacement l'un de l'autre, chacun desdits canaux étant en communication avec la sonde d'aspiration trachéale et susceptible de véhiculer l'air respiré, ainsi qu'en communication avec un canal (6), de forme tubulaire, pour l'évacuation des expectorations vers l'extérieur, l'extrémité interne frontale dudit canal étant fermée tandis qu'avec son espace intérieur de canal vient en ajustement d'appui une trompe (7), de forme tubulaire, susceptible de pivoter et/ou de se déformer par elle-même grâce à la force de gravitation, réalisée en un matériau de flexibilité élevée ou ayant l'élasticité d'un ressort, ladite trompe (7) étant de conformation telle qu'elle s'étend de façon transversale par rapport au canal (6) pour l'évacuation des expectorations et telle que son extrémité libre est séparée mais jusqu'à s'approcher de la paroi interne de l'enveloppe (1).
- **caractérisé en ce que** la trompe (7) est librement déformable en courbure et **en ce qu'**elle est conformée en rotation libre et étanche autour de l'axe longitudinal du canal (6), avec son extrémité côté canal engagée sur le canal (6) pour l'évacuation des expectorations,
- **caractérisé en ce que** la trompe (7) débouche par son extrémité côté canal dans une douille en forme de pot (26) et est libre en rotation, au moyen de la douille engagée sur le canal (6) pour l'évacuation des expectorations, autour de l'axe longitudinal médian de celui-ci,
- **caractérisé en ce que** les canaux (6, 5) pour l'évacuation des expectorations et pour véhiculer l'air respiré sont conjointement conformés, par rapport à l'enveloppe (1), en étant susceptibles de pivotement ou de rotation,
- **caractérisé en ce que** la trompe (7) repose, par son extrémité libre, sur la paroi interne de l'enveloppe (1) et présente, à son extrémité dirigée vers ladite paroi interne, des ouvertures de passage (25) ou analogues pour l'admission des expectorations dans la trompe (7),
- **caractérisé en ce que** la trompe (7) porte à l'extérieur de son extrémité libre un corps, par exemple un corps annulaire (33) de poids spécifique élevé et **en ce que** la trompe (7), conjointement avec le corps de poids (33) est susceptible d'être amenée et maintenue dans l'enveloppe (1), en ajustement radial par rapport au canal (6) pour l'évacuation des expectorations,
- **caractérisé en ce que** sur le canal (6) pour l'évacuation des expectorations est ajustée une pompe (13) par son refoulement (13"'), tandis que l'admission (13'''') de ladite pompe est en communication avec la trompe (7),
- **caractérisé en ce que** la pompe (13) est une pompe à piston ou à membrane et **en ce que** le piston (13') ou la membrane de la pompe (13) sont susceptibles d'être amenés, par le biais de la pression de respiration, depuis la position de repos jusqu'en position de pompage des expectorations et sont susceptibles d'être ramenés automatiquement en position de repos par le biais d'une force élastique et/ou de gravitation en cas d'absence d'air respiré,
- **caractérisé en ce que** la force élastique est susceptible d'être engendrée par un corps formant plaque, déformable et en un matériau ayant l'élasticité d'un ressort, ledit corps formant plaque étant fixé à l'enveloppe (1) ou au canal (6) pour l'évacuation des expectorations et étant solidaire par le biais d'un organe d'actionnement avec le piston ou la membrane de la pompe,
- **caractérisé en ce que** la force élastique est susceptible d'être appliquée par le biais d'un corps rigide formant plaque (14) disposé le long d'un manchon à profil ondulé (15), en un matériau ayant l'élasticité d'un ressort, et **en ce que** le corps formant plaque (14) est relié, par le biais d'un organe d'actionnement, au piston (13') ou à la membrane de la pompe (13),
- **caractérisé en ce que** le canal (6) pour l'évacuation des expectorations présente, afin d'empêcher des reflux indésirables d'expectoration, une barrière de reflux, avec une action de barrière ajustable dans le sens du reflux (8).
